# EUROPEAN PATENT APPLICATION

(11) **EP 1 795 228 A1**
(43) Date of publication of application: **13.06.2007**
(21) Application number: 06124704.5
(22) Date of filing: 24.11.2006
(51) Int. Cl.: A61N 5/06

(54) **Body tanning bed**

(30) Priority: 30.11.2005 IT TV20050186
(71) Applicant: Quadra Medical s.r.l., 36060 Romano d'Ezzelino VI (IT)
(72) Inventor: Burato, Flavio, 36060, Romano d'Ezzelino VI (IT); Durighel, Fabio, 31044, Montebelluna TV (IT)
(74) Representative: Modiano, Micaela Nadia

(57) **Abstract**

A body tanning bed, comprising a bench (3) provided with a plurality of first low- and/or high-pressure sources (6) on which a user (2) lies and above which a canopy (4) is arranged which is provided with a plurality of second low- and/or high-pressure sources (7); in the bed (1), the distance (D1, D2) between the first and second sources (6, 7) decreases from the head (5) toward the feet (8) of the user (2), so as to improve leg tanning. Moreover, on the canopy (4) there is, at least at the head (5) of the user (2), at least one nozzle (9) for spraying a self-tanning and/or cosmetic product at least onto the face of the user (2).

## Description

Various types of bed used to tan the body are currently known; therefore, for example, it is known to provide beds constituted by a usually horizontal bench on which the person who wishes to tan lies; such bench is pivoted, along a longitudinal side, to a canopy which usually is lowered onto the person so as to surround him or her completely along the length of his or her body.

UV sources are associated at the bench and at the canopy: for example, low- and/or high-pressure sources arranged inside the canopy and low- and/or high-pressure sources arranged at the bench.

Ventilation systems are also provided which are usually used to cool the lamps and/or ventilate the body.

In a transverse cross-section, the arrangement of the sources usually follows the shape of the bench and of the internal surface of the canopy, and the sources are arranged at mutually fixed distances along a longitudinal axis.

The main drawback that can be observed in this type of known method is that the tanning achieved is uneven, since there are parts of the body which tan differently for equal exposure times, and therefore it is necessary to either increase the number of sessions, in order to avoid having parts of the body which are not sufficiently tanned, or have specific sessions for tanning just one or more parts of the body, so that it is possible to achieve complete uniformity of the tan over the entire body.

Uneven body tanning can be due both to a problem of the anatomical shape of the body of the user and to a technical problem arising from the use of the bed.

In the first case, blood circulation is in fact different in the leg region with respect to the trunk region, whereas in the second case a reduced ultraviolet ray emission is observed, over time, at the ends of the tubes that constitute the sources.

The aim of the present invention is to solve the above-mentioned problems, eliminating the drawbacks of the cited background art, by providing a device which allows to achieve uniform tanning of the various parts of the body and therefore of the legs, trunk and face for an equal exposure to ultraviolet rays.

Within this aim, an object of the invention is to provide a device which allows the user to achieve said uniform tanning by means of a device which is structurally simple and has low manufacturing costs.

This aim and this and other objects, which will become better apparent hereinafter, are achieved by a body tanning bed, which comprises a bench provided with a plurality of first low- and/or high-pressure sources on which a user lies and above which a canopy is arranged which is provided with a plurality of second low- and/or high-pressure sources, characterized in that the distance between said first and second sources decreases from the head toward the feet of the user and in that on said canopy there is, at least at the head of the user, at least one nozzle for spraying a self-tanning and/or cosmetic product at least onto the face of the user.

In a variant form of the invention, a body tanning stand-up booth is provided, which comprises a back, which is provided with a plurality of first low- and/or high-pressure sources on which a user rests, in use, and which can be closed at the front by means of a door provided with a plurality of second low- and/or high-pressure sources, characterized in that the distance between said first and second sources decreases from the head toward the feet of the user and in that on said door there is, at least at the head of the user, at least one nozzle for spraying a self-tanning and/or cosmetic product at least onto the face of the user.

Further characteristics and advantages of the invention will become better apparent from the following detailed description of a particular but not exclusive embodiment thereof, illustrated by way of non-limiting example in the accompanying drawings, wherein:

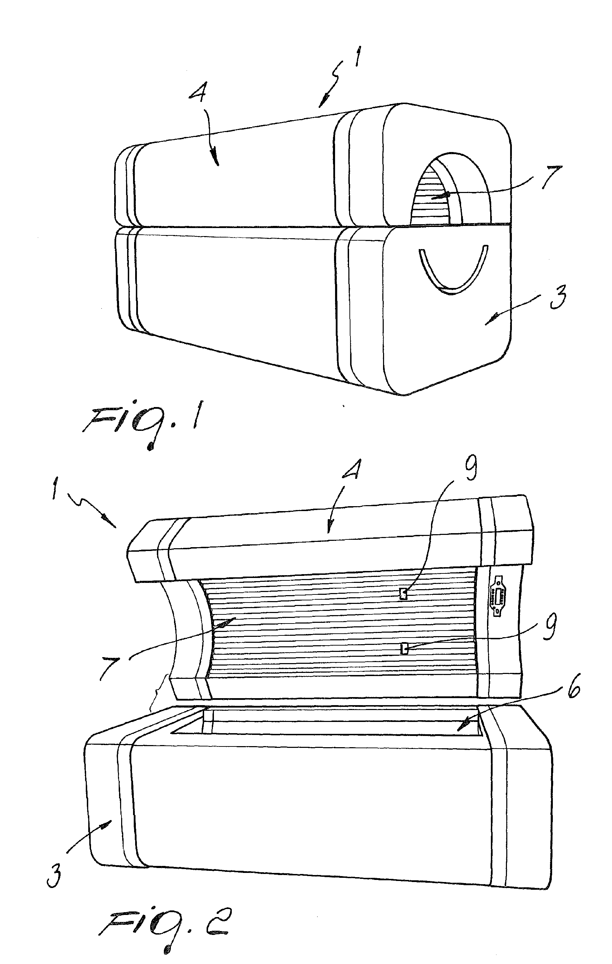

Figure 1 is a front perspective view of the bed in the condition in which the canopy is closed;
Figure 2 is a view, similar to Figure 1, of the bed with the canopy open;
Figure 3 is a schematic side view of the arrangement of the user inside the bed and of the various distances between the first and second sources;
Figure 4 is a schematic view, taken transversely with respect to the bed, of the arrangement of the nozzles for spraying a self-tanning product onto the face of the user;
Figure 5 is a schematic side view of a stand-up booth provided according to the same teachings.

In the exemplary embodiments that follow, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

With reference to the figures, the reference numeral 1 designates a bed for tanning the body 2 of a user.

The bed is constituted by a bench 3, which has a preferably rectangular plan shape which is open at the ends; a canopy 4 is pivoted along a longitudinal side of said bench, has a preferably rectangular plan shape and is open at its ends.

A plurality of first low- and/or high-pressure UV sources 6, such as UV lamps, advantageously approximately 1.8 meters long, is associated with the surface of the bench 3 in the region below the body of the user, lying in use thereon, except for a region 5 of the nape, or head region.

A plurality of second low- and/or high-pressure UV sources 7, advantageously approximately 2 meters long, is associated at the internal surface of the canopy 4, which lies above the entire body of the user.

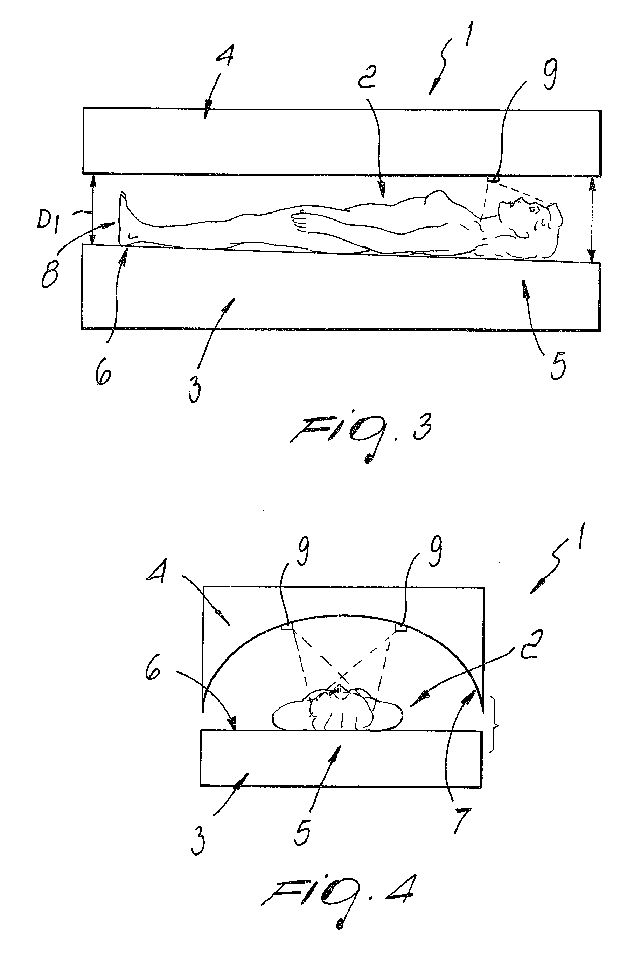

As shown in Figure 3, the bed 1 has a distance between the plurality of first low- and/or high-pressure sources 6 such as UV-emitting fluorescent, pressure lamps for tanning, and the plurality of second low- and/or high-pressure sources 7 which is not constant, i.e. varies along the entire body of the user; as shown in Figure 3, the distance, designated by the reference sign D1, between the first and second sources approximately starting from, or extending at the foot region 8 is different from the distance, designated by the reference sign D2, between the first and second sources approximately at the head region 5 of the user's head.

In the particular embodiment shown in Figure 3, the distance D1 is shorter than the distance D2 and therefore the first and second sources are mutually closer at the foot region 8, so that during tanning the legs receive a more intensive treatment and therefore a better tan.

The greater proximity of the second sources to the legs therefore compensates both a lower UV emission at the end of the tubes that constitute the second sources and the fact that there is a lower blood circulation in the legs with respect to the trunk.

In the embodiment shown in Figure 3, the region where the body rests on the bench is assumed to be in practice inclined; as an alternative, this resting region might be flat and the region where the second sources 7 are arranged might instead be inclined.

The bed further has, at least at a region thereof that lies above the head of the user, at least one nozzle 9 for spraying a self-tanning and/or cosmetic product onto the face of the user.

As an alternative, the at least one nozzle 9 can be provided at the region of the bed that lies above the neck and/or trunk and/or pelvis and/or legs and/or feet of the user.

In the particular embodiment shown in Figure 4, the nozzles are two and are arranged in a region of the bed which lies laterally with respect to its region above the head of the user.

The session can take place as follows: after a few minutes, preferably no less than four minutes of UV session, the body/face ventilation system is stopped, in order to avoid interfering with the subsequent vaporization of the self-tanning and/or cosmetic liquid; the self-tanning and/or cosmetic lotion is then sprayed and body/face ventilation is restarted; of course, the UV session is never interrupted; moreover, spraying can be repeated several times.

It has thus been found that the invention has achieved the intended aim and objects, a body tanning bed having been obtained which, thanks to the inclined arrangement of the first and second sources and of the presence of the nozzles for spraying the self-tanning and/or cosmetic product, allows to achieve uniform and optimum tanning of the entire body of the user for an equal number of sessions.

The combination between the tanning provided by UV sources and sources of self-tanning and/or cosmetic liquid in fact allows to achieve the following advantages in a single session: immediate tanning, provided by the self-tanning product, with a considerable advantage for the user because said user no longer needs to expose him or herself to UV sources, and extremely prolonged and uniform tanning thanks to the effect of the UV sources.

Moreover, the tanning achieved by the combination of UV and self-tanning product has superior results with respect to a single UV session or to a single self-tanning session, since the UV allows widening of the pores of the skin and therefore deeper and more uniform absorption of the sprayed self-tanning lotion.

The invention thus conceived is of course susceptible of numerous modifications and variations, all of which are within the scope of the appended claims.

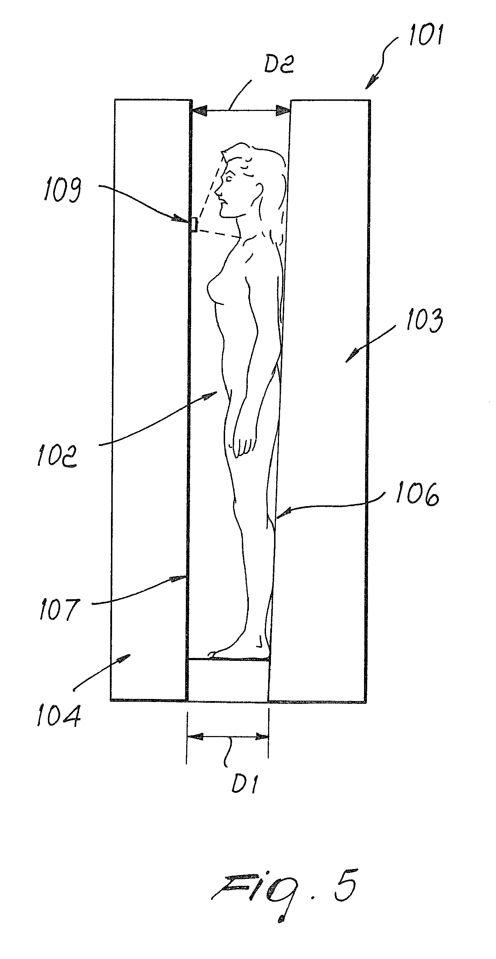

Thus, for example, with reference to Figure 5, according to the same inventive concept it is possible to provide a stand-up booth 101 for tanning the body 102 of the user which comprises a back 103 provided with a plurality of first low- and/or high-pressure sources 106 on which the user rests.

At the front, the back can be closed by means of a door 104 onto which a plurality of second low- and/or high-pressure sources 107 are applied; in this embodiment also, the distance between said first and second sources decreases from the head toward the feet of the user, and on the door 104 there is, at least at the head of the user, at least one nozzle 109 for spraying a self-tanning and/or cosmetic product at least onto the face of the user.

Correct use of the booth 101 requires the user to tend to rest with his head, back, buttocks, calves and heels against the back 103, so as to keep the body 102 at a preset distance from the second sources 107.

The materials used, as well as the dimensions that constitute the individual components of the invention, may of course be more pertinent according to specific requirements.

The various means for performing certain different functions need not certainly coexist only in the illustrated embodiment but can be present per se in many embodiments, including ones that are not illustrated.

The characteristics indicated as advantageous, convenient or the like may also be omitted or be replaced with equivalents.

The disclosures in Italian Patent Application No. TV2005A000186 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A body tanning bed, comprising a bench (3) provided with a plurality of first low- and/or high-pressure sources (6) on which a user (2) may lie in use and above which a canopy (4) is arranged which is provided with a plurality of second low- and/or high-pressure sources (7), **characterized in that** the distance (D1, D2) between said first and second sources (6, 7) decreases from the head (5) toward the feet (8) of the user (2) and **in that** on said canopy (4) there is, at least at the head (5) of the user, at least one nozzle (9) for spraying a self-tanning and/or cosmetic product at least onto the face of the user (2).

2. The bed according to claim 1, **characterized in that** the distance (D1, D2) between said pluralities of first and second low- and/or high-pressure sources (6, 7) varies along the entire body of the user (2).

3. The bed according to claims 1 and 2, **characterized in that** the distance (D1), between said first and second sources (6, 7) approximately starting from the foot region (8) is different from the distance (D2), between said first and second sources (6, 7) approximately at the region (5) of the head of the user (2).

4. The bed according to claims 1 and 3, **characterized in that** said distance at the foot region (D1) is shorter than said distance at the head region (D2) and said first and second sources (6, 7) are mutually closer at the foot region (8).

5. The bed according to one or more of the preceding claims, **characterized in that** the region for supporting the body of the user (2), above said bench (3), is inclined.

6. The bed according to one or more of the preceding claims, **characterized in that** the region of said canopy (4) which lies above the body of the user (2) is inclined.

7. The bed according to one or more of the preceding claims, **characterized in that** it has, at least at the region that lies above the head of the user (2), at least one nozzle (9) for spraying a self-tanning and/or cosmetic product onto the face of the user (2).

8. The bed according to one or more of the preceding claims, **characterized in that** it has, at the region that lies above the neck and/or trunk and/or pelvis and/or legs and/or feet of the user (2), at least one nozzle (9) for spraying a self-tanning and/or cosmetic product onto the body of the user (2).

9. The bed according to one or more of the preceding claims, **characterized in that** it has, at the region that lies above the head of the user (2), two nozzles (9) for spraying a self-tanning and/or cosmetic product onto the face of the user, said two nozzles (9) being arranged laterally with respect to the head of the user (2).

10. A body tanning bed, comprising a bench (3) provided with a plurality of first low- and/or high-pressure sources (6) on which a user (2) lies and above which a canopy (4) is arranged, said canopy (4) having a plurality of second low- and/or high-pressure sources (7), **characterized in that** the distance (D1, D2) between said first and second sources (6, 7) decreases from the head (5) toward the feet (8) of the user (2).

11. A body tanning stand-up booth, comprising a back (103), provided with a plurality of first low- and/or high-pressure sources (106) on which a user (102) rests, said back (103) being closable at the front by means of a door (104) provided with a plurality of second low- and/or high-pressure sources (107), **characterized in that** the distance (D1, D2) between said first and second sources (106, 107) decreases from the head toward the feet of the user (102) and **in that** on said door (104) there is, at least at the head of the user, at least one nozzle (109) for spraying a self-tanning and/or cosmetic product at least onto the face of the user (102).
